# FASCICULE DE BREVET EUROPEEN

(11) **EP 1 413 293 B1**
(45) Date de publication et mention de la délivrance du brevet: **05.09.2007**
(21) Numéro de dépôt: 03292572.9
(22) Date de dépôt: 15.10.2003
(51) Int. Cl.: A61K 9/06

(54) **Pommade conçue pour assurer la disparition des champignons**
Salbe zum Bestreiten von Pilzen
Ointment for combatting fungi

(30) Priorité: 24.10.2002 FR 0213330
(43) Date de publication de la demande: 28.04.2004
(73) Titulaire: Aliaga, Maria Yolanda, 75015 Paris (FR)
(72) Inventeur: Aliaga, Maria Yolanda, 75015 Paris (FR)

(56) Documents cités:
- DE-U- 20 201 016
- FR-A- 2 743 720
- DATABASE BIOSIS [en ligne] BIOSCIENCES INFORMATION SERVICE, PHILADELPHIA, PA, US; AL-ANI FK ET AL.: "Ringworm infection in cattle and horses in Jordan" Database accession no. prev200200316729 XP002246348 & ACTA VETERINARIA BRNO, vol. 71, no. 1, mars 2002 (2002-03), pages 55-60,
- DATABASE WPI Week 199321 Derwent Publications Ltd., London, GB; AN 1993-173514 XP002246349 & RO 104 280 B (INST. POLITEHNIC TIMISOARA), 15 décembre 1993 (1993-12-15)
- DATABASE WPI Week 199809 Derwent Publications Ltd., London, GB; AN 1998-099209 XP002246350 & RU 2 083 224 C (PODCHAINOV), 10 juillet 1997 (1997-07-10)
- DATABASE WPI Week 200162 Derwent Publications Ltd., London, GB; AN 2001-550459 XP002246351 & CN 1 301 537 A (FAN), 4 juillet 2001 (2001-07-04)
- DATABASE WPI Week 200027 Derwent Publications Ltd., London, GB; AN 2000-315826 XP002246352 & RU 2 130 772 C (TSAMAKS CO LTD), 27 mai 1999 (1999-05-27)

## Description

La présente invention concerne une pommade antimycosique préconisée pour traiter les mycoses et assurer la disparition progressive.
La pommade est de consistence visqueuse, couleur sombre.
Le mycoses, affection parasitaire provoquée par des champignons microscopique (chez l'homme et animal) ensemble des modifications que subissent la peau ou les tissus sous-jacent à la suite de l'humidité.
Seule l'élimination des déchets que le tissus cutanés n'ont pas réussi à évacuer permet d'arrêter la croissance fonfistatique.
La pommade par un apport local d'éléments appropiés, tels que soufre (fleur de iode, acide salicylique ainsi que les huiles essentielles ayant un pouvoir spécifiquement purifiant désinfectant et cicatrisant, satisfait a ses exigences par :
- Sa grande efficacité due à un choix et à un dosage judicieux de ses composants, à une concentration et à une haute diffusibilité permet des résultats satisfaisants.
- Son effet exfoliant doux et sans aucun effet traumatisant, permet un désencrassement constant jusqu'a la disparition parasitaire.
- Ses propriétés cicatrisants, les huiles essentielles , iode et la vaseline diluent et éliminent les déchets cutanés provoque par des mycetes.
- Ses propriétés régénérants équilibrantes, les tissus cutanés alléges de leurs déchets et mieux irrigués; retrouvent élasticité et bien être.

Le document Acta Veterinaria Brno, 71(1), p. 55-60, 2002 décrit l'utilisation d'acide salicylique, acide benzoïque, souffre et iode dans de la vaseline pour le traitement du bétail et des chevaux.

Utilisation- La composition antimycosique peut-être utilisé en médicine et les produits de beauté avec les propriétés anti-bactériennes. Le lineament à l'effet unique de traitement sur la maladie de la peau provoque par des mycètes.
Avantage - La composition peut-être employée, comme solide liquide et spray. Bas en coût.
- L'application de la présente invention est la suivante :
   Dans une bassine remplie d'eau tiède et de savon liquide de (préférence), mettre les pieds et les laisser tremper un bon moment. Les sécher avec une serviette personnalisée, pour les parties atteintes, puis avec un séche-cheveux enlever toute trace d'humidité. Etaler la pommade entre les orteils puis passer entre ceux-ci, une compresse en accordéon et laisser.
   Exemple - doigts, appliquer la pommade et couvrir avec un morceau de compresse puis mettre un doigtier pour la journée, la nuit, de préférence sans doigtier.
- Pour le corps, étaler une fine couche.
Après les soins, bien se laver les mains pour éviter toute propagation.

POUR LE CORPS, une application par jour.
POUR LES DOIGTS, deux fois par jour matin et soir
PIEDS, ENTRE-ORTEILS, une application par jour matin ou soir jusqu'à la disparition de l'affection.

DUREE d'application, deux à trois semaines, varies selon l'état de le peau et l'étendue.

| | | | |
|---|---|---|---|
| - vaseline | entre 20 gr. | 45 gr. | et 92 gr. |
| - soufre (fleur de) | entre 1 gr. | 1,5gr. | et 3 gr. |
| - acide salicylique | entre 1/2gr. | 1 gr. | et 2 gr. |
| - iode | entre 10 gttes. | 20gttes. | et 30 gttes. |
| - huiles essentielles | | | |
| cannelle de ceylan | entre 1 gtte. | 2 gttes. | et 3 gttes. |
| citron entre | 1 gtte. | 2 gttes. | et 3 gttes. |
| genièvre | entre 1 gtte. | 2 gttes | et 3 gttes. |
| menthe poivrée | entre 1 gtte | 2 gttes. | et 3 gttes. |
| patchouli | entre 1 gtte. | 2 gttes. | et 3 gttes. |
| sarriette de montagne | entre 1 gtte. | 2 gttes. | et 3 gttes. |
| santal | entre 1 gtte. | 2 gttes. | et 3 gttes. |
| laurier | entre 1 gtte. | 2 gttes. | et 3 gttes. |

| | |
|---|---|
| - vaseline | entre 65 gr et 80 gr |
| - acide salicylique | entre 1 gr et 1,5gr |
| - iode | entre 15gttes et 25 gttes. |
| - alcool iodée | entre 7 gttes et 15 gttes. |
| - HE | |
| cannelle de ceylan | entre 2 gttes et 4 gttes. |
| citron | entre 3 gttes et 4 gttes. |
| genièvre | entre 2 gttes et 4 gttes. |
| menthe poivrée | entre 3 gttes et 4 gttes. |
| patchouli | entre 2 gttes et 4 gttes. |
| sarriette de montagne | entre 3 gtte et 4 gttes. |
| santal | entre 2 gttes et 4 gttes. |
| laurier | entre 2 gttes et 4 gttes. |

### EXEMPLE DE PREPARATION

Dans un verre mélangeur en verre ou en inox
- déposer la vaseline
- ajouter le soufre , l'acide salicylique préalablement tritures dans les HE. ensuite l'iode, mélanger le tout jusqu'à complète hogénité des ingrédients.
- conditionner immédiatement et entreposer à l'abri des rayons solaires dans un pot de verre de préférence foncée et dont le couvercle est à vis.

L'objet de l'invention ayant été décrit il est déclaré que ce qui constitue l'essentiel de l'invention est ce qui est concrétisé dans les revendications qui suivent.

## Revendications

1. Pommade antifongique à l'effet unique de traitement sur la maladie de la peau provoque par Des mycètes ayant un pouvoir spécifiquement désinfectant, purifiant et cicatrisant
- vaseline
- soufre (fleur de )
- acide salicylique
- iode
**caractérisée par** la composition qualitative et quantitative :
| | | | | |
|---|---|---|---|---|
| - vaseline | entre | 20 gr | et | 45 gr |
| - soufre (fleur de ) | entre | 1 gr | et | 1,5 gr |
| - acide salicylique | entre | ½ gr | et | 1 gr |
| - iode | entre | 10 gttes | et | 20 gttes |
huiles essentielles choisies parmi :
| | | | | |
|---|---|---|---|---|
| - cannelle de ceylan | entre | 1 gtte | et | 2 gttes |
| - citron | entre | 1 gtte | et | 2 gttes |
| - genièvre | entre | 1 gtte | et | 2 gttes |
| - menthe poivre | entre | 1 gtte | et | 2 gttes |
| - patchouli | entre | 1 gtte | et | 2 gttes |
| - sarriette de montagne | entre | 1 gtte | et | 2 gttes |
| - santal | entre | 1 gtte | et | 2 gttes |
| - laurier | entre | 1 gtte | et | 2 gttes |

2. Pommade selon la revendication 1, dans laquelle on peut utiliser :
- acide lactique
- borax
- talc, comme agent remplissant.

## Claims

1. Single effect anti-fungal ointment for treatment of skin disease caused by fungi, with a specifically disinfecting, purifying and cicatrizing capacity
- Vaselines
- Sulphur (flower of)
- Salicylic acid
- Iodine
**Characterised by** the qualitative and quantitative composition:
| | | | | |
|---|---|---|---|---|
| - Vaseline | between | 20 g. | and | 45 g. |
| - sulphur (flower of) | between | 1 g. | and | 1.5 g. |
| - salicylic acid | between | 0.5 g. | and | 1 g. |
| - iodine | between | 10 drops | and | 20 drops |
Essential oils chosen from:
| | | | | |
|---|---|---|---|---|
| - cinnamon | between | 1 drop | and | 2 drops |
| - lemon | between | 1 drop | and. | 2 drops |
| - juniper | between | 1 drop | and | 2 drops |
| - peppermint | between | 1 drop | and | 2 drops |
| - patchouli | between | 1 drop | and | 2 drops |
| - winter savoury | between | 1 drop | and | 2 drops |
| - sandal | between | 1 drop | and | 2 drops |
| - laurel | between | 1 drop | and | 2 drops |

2. Ointment as per claim 1, in which the following may be used:
- lactic acid
- borax
- talc, as filling agent

## Patentansprüche

1. Antimykotikum-Salbe, die eine besonders desinfizierende, reinigende und selbstheilende Fähigkeit hat und eine einzigartige Wirkung für die Behandlung von durch Myzeten hervorgerufe Hautkrankheiten.
- Vaseline
- Schwefel (Schwefelblüte)
- Salicylsäure
- Jod **Gekennzeichnet durch** die qualitative und quantitative Zusammensetzung:
| | | | |
|---|---|---|---|
| - Vaseline | zwischen | 20 gr. und | 45 gr. |
| - Schwefel (Schwefelblüte) | zwischen | 1 gr. und | 1,5 gr. |
| - Salicylsäure | zwischen | 1/2 gr. und | 1 gr. |
| - Jod | zwischen | 10 Tropfen | und 20 Tropfen |
Ätherische Öle und zwar:
| | | | | |
|---|---|---|---|---|
| - Cylon-Zimt | zwischen | 1 Tropfen | und | 2 Tropfen |
| - Zitrone | zwischen | 1 Tropfen | und | 2 Tropfen |
| - Wacholder | zwischen | 1 Tropfen | und | 2 Tropfen |
| - Pfefferminze | zwischen | 1 Tropfen | und | 2 Tropfen |
| - Patschuli | zwischen | 1 Tropfen | und | 2 Tropfen |
| - Bergminze | zwischen | 1 Tropfen | und | 2 Tropfen |
| - Sandelholz | zwischen | 1 Tropfen | und | 2 Tropfen |
| - Lorbeer | zwischen | 1 Tropfen | und | 2 Tropfen |

2. Salbe gemäß Anspruch 1, in der Folgendes eingesetzt werden kann:
- Milchsäure
- Natriumtetraborat
- Talk als Füllmittel.
